# EUROPEAN PATENT SPECIFICATION

(11) **EP 0 386 117 B1**
(45) Date of publication and mention of the grant of the patent: **07.01.1999**
(21) Application number: 88910336.2
(22) Date of filing: 21.10.1988
(51) Int. Cl.: A61K 45/06, A61K 31/135, A61K 31/405, A61K 31/00, A61K 33/00

(54) **TREATING PREMENSTRUAL OR LATE LUTEAL PHASE SYNDROME**
BEHANDLUNG DES PRÄMENSTRUELLEN ODER SPÄTEN LUTEALSPHASE-SYNDROMS
TRAITEMENT DU SYNDROME DE PHASE LUTEALE TARDIVE OU PREMENSTRUELLE

(30) Priority: 22.10.1987 US 111771; 15.09.1988 US 244944
(43) Date of publication of application: 12.09.1990
(73) Proprietor: MASSACHUSETTS INSTITUTE OF TECHNOLOGY, Cambridge, MA 02139 (US)
(72) Inventor: WURTMAN, Richard, J., Boston, MA 02216 (US); WURTMAN, Judith, J., Boston, MA 02116 (US)
(74) Representative: Kirkham, Nicholas Andrew
(86) International application number: US8803709
(87) International publication number: WO8903692

(56) References cited:
- EP-A- 0 123 469
- EP-A- 0 253 146
- GB-A- 2 129 299
- US-A- 4 210 637
- US-A- 4 309 445
- Wurtman, J.J. et al.: LIFE SCIENCES, vol. 24, no. 10, 1979, pages 895-904
- Diagnostic and Statitical Manual of Mental Disorders, DSM-III-R, American Psychiatric Association, Washington, D.C., U.S.A., 1987
- AMERICAN JOURNAL OF PSYCHIATRY, Vol. 141, No. 2, February 1984, (Washington), DAVID R. RUBINOW & PETER ROY-BYRNE, "Premenstrual Syndromes: Overview from a Methodologic Perspective", pages 163-172.
- DRUGS, Vol. 24, 1982, (Sydney), P.M.S. O'BRIEN, "The Premenstrual Syndrome: A Review of the Present Status of Therapy", pages 140-151.
- ARCHIVES INTERNATIONALES DE PHARMACODYNAMIE ET DE THERAPIE, Vol. 248, No. 1, 1980, G.L. DIGGORY et al., "Behavioural and Neurochemical Properties of 1-[1-([Indol-3-yl]-methyl)piperid-4-yl]-3-benzoylurea (Wy 25093) in Rodents", pages 86-104.
- OBSTETRICS AND GYNECOLOGY, Vol. 70, No. 4, October 1987, (New York), A.J. RAPKIN et al., "Whole-Blood Serotonin in Premenstrual Syndrome", pages 533-537.
- JOURNAL OF CLINICAL PSYCHIATRY, Vol. 49, No. 8, (Suppl.), August 1988, (Memphis), J.J. WURTMAN, "Carbohydrate Craving, Mood Changes and Obesity", pages 37-39.

## Description

### Background

Each month, for a few days prior to the onset of menstruation, many millions of otherwise-healthy American women develop symptoms of disturbed mood and appetite that can be strikingly similar to those reported by patients with Seasonal Affective Disorder (SAD), carbohydrate-craving obesity, or the non-anorexic variants of bulimia. This syndrome was first termed "premenstrual tension" by R. T. Frank in 1931 and is a very common phenomenon. According to Guy Abraham of UCLA, "...of every ten patients to walk into a gynecologist's office, three or four will suffer from premenstrual tension...", and in some the symptoms will be of such severity as to include attempts at suicide. Current Progress in Obstetrics and Gynecology, 3:5-39 (1980).

Initial descriptions of the Premenstrual Syndrome (PMS) focused on its association with "nervous tension", headache, and weight gain. The weight gain observed was initially attributed to excessive retention of salt and water, which does indeed occur in some PMS patients. However, it soon became evident that it was also a consequence of the widespread tendency of PMS individuals to crave and overconsume carbohydrates, particularly foods with a sweet taste. PMS is also now referred to as late luteal phase syndrome. D.N.S. III, Revised, American Psychiatric Association (1987).

There have been numerous suggestions made about the etiology of PMS. For example, some hypothesized that it was caused by a uterine toxin. Others suggested its cause was overconsumption of sweets, which was presumably followed by excessive insulin secretion, hypoglycemia, and inadequate brain glucose and resulted in the often observed depression and anxiety. It has also been postulated that the behavioral symptoms result from the tissue edema often observed and that the psychological changes result from feelings of loss or the social complexities generated by the discomforts of menstruation.

However, none of these theories has been substantiated: PMS can persist after hysterectomy and, hence, uterine toxins cannot be its cause; the hyperinsulinism of PMS is not associated with low blood glucose levels, and is probably the consequence of a behavioral aberration (i.e., the tendency of premenstrual women to choose high-carbohydrate diets, which potentiate insulin secretion)--rather than the cause; the mood and appetitive changes of PMS are poorly correlated with the tissue swelling; and subhuman primates who are presumably exempt from the psychodynamic or social complexities of human life, also exhibit characteristic behavioral changes premenstrually.

There have been many treatments suggested for overcoming or reducing the symptoms of PMS. These include carbohydrate-free diets, vitamin supplements, ovarian hormones, detoxifying agents, irradiation of the ovaries and pituitary, and use of diuretics. These approaches have all had limited success, however, and a means of treating the mood and appetite disturbances commonly experienced on a recurring basis by a large number of women would be of great benefit.

Am. J. Psychiatry 141:163-172, 1984 reviews the available evidence regarding the nature, cause, and treatment of the premenstrual syndromes. They attribute the contradictory results of various studies and the current theoretical confusion in the area to the failure of investigators to carefully define the syndromes, formulate a set of answerable questions, and select a homogeneous population before initiating their studies. The relationship between premenstrual syndromes and major psychiatric disorders, as well as the clinical and theoretical relevance of the menstrual cycle to major psychiatric disorders, is discussed. The authors offer recommendations to both investigators and clinicians for more careful observation and documentation of the relationship between mood disorders and the menstrual cycle.

Drugs 24: 140-151 (1982) discloses that treatment of the premenstrual syndrome is complicated by many factors, but principally by its unknown aetiology. In addition, diagnosis, definition and symptom evaluation methods are unclear. The multitudinous studies of treatment regimens have been largely inconclusive; this is partly due to difficulties in numerically evaluating the symptoms, but more to the lack of appreciation of the marked placebo effect which has been estimated as being up to 50%. The majority of studies have been open studies, and therefore interpretation of the results have been almost impossible. The end result is that great claims have been made for a large number of therapeutic agents on ill-founded evidence. Some of the drugs used in the treatment of the premenstrual syndrome, however, have been better evaluated than others, although even with those studied more extensively results have often been variable. Thus, hormonal agents such as progestagens and oral contraceptives, diuretics, pyridoxine, bromocriptine and danazol have been effective in some studies but not universally so. The latter 2 agents seem to be effective in relieving breast symptoms, but have only a limited effect on other symptoms. It is therefore important to realise that one drug does not cure all patients or all symptoms, although it is often claimed that this is the case. It has been suggested that more than 40% of women suffer from premenstrual syndrome. Greater awareness of the problem, both by patients and doctors, necessitates a more rational approach to therapy.

GB-A-2,129,299 discloses pharmaceutical compositions for use as antidepressants, comprise L-tryptophan and a monoamine oxidase inhibitor e.g. phenelzine or tranylcypromine the L-tryptophan and monoamine oxidase inhibitor being present at the ratio of not more than 1.5g of L-tryptophan per daily dose of the monoamine oxidase inhibitor. The compositions may also contain folic acid, ascorbic acid, pyridoxine, thiamine, riboflavin, nicotinic acid or nicotinameide.

Obstet. Gynecol. 70:533, 1987 discloses that whole-blood serotonin levels in 14 subjects with well documented premenstrual syndrome and 13 age-matched controls were compared. Serotonin levels of premenstrual syndrome subjects were significantly lower during the last ten days of the menstrual cycle. No significant differences were noted in levels of serum estradiol and progesterone. Decreased serotonin is known to be associated with depression in humans, and nonhuman primates have exhibited abnormal behavioural profiles when given serotonin antagonists. The present observation suggests that the physiologic basis of premenstrual syndrome involves an alteration in serotonin metabolism.

Arch. Int. Pharmacodyn. 248, 86-104 (1980) discloses Wy 25093, a novel, selective and potent inhibitor of the neuronal 5-hydroxytryptamine (5-HT) uptake process in vitro and in vivo. The compound was more potent than clomipramine and fluoxetine as an inhibitor of 5-HT uptake in vitro and did not significantly inhibit catecholamine uptake. In addition, Wy 25093 potentiated the behavioural syndrome induced by 5-hydroxytryptophan (5-HTP) and antagonised the hyperactivity produced by p-chloroamphetamine (P-CA). Wy 25093 antagonised the (P-CA)-induced depletion of 5-HT in rat brain and reduced the probenecid-induced increase in rat brain 5-hydroxyindole-3-acetic acid (5-HIAA). Acute administration of the agent to rats resulted in reduced 5-HIAA levels without affecting 5-HT; chronic treatment with the compound produced decreases in the levels of both 5-HIAA and 5-HT. It is concluded that Wy 25093 is a selective and potent inhibitor of the neuronal re-uptake process for 5-HT both in vitro and in vivo, and may possess potential antidepressant activity.

EP-A-0,123,469 discloses the use of fluoxetine as an anti-anxiety agent. Fluoxetine, norfluoxetine, and pharmaceutically acceptable salts thereof are anti-anxiety agents.

US-A-4,210,637 discloses a composition, comprising tryptophan and an insulin-releasing carbohydrate, is administered to an animal prior to consuming food; this suppresses total caloric intake while having only a small effect on protein intake.

US-A-4,309,445 discloses compositions containing controlled amounts of d-fenfluramine are administered to block the intermittent carbohydrate cravings without necessarily suppressing other food intakes.

Life Sciences, Vol. 24, pp. 895-904 discloses drugs that enhance central serotoninergic transmission diminish elective carbohydrate consumption by rats. The authors previously showed (Science 198:1178, 1977) that fenfluramine or fluoxetine, drugs thought to enhance serotoninergic transmission, selectively decrease the rat's consumption of carbohydrates, without affecting protein intake, when animals are given simultaneous access to diets differing in protein (5% vs 45%) and carbohydrate contents; in contrast, d-amphetamine lacks this selective effect. Present studies affirm this relationship using another serotoninergic drug, MK-212, and show that the suppression of carbohydrate intake occurs whether the test diets contain variable amounts of protein, or carbohydrates, or of both nutrients. Evidence is presented that rats given diet mixtures containing various proportions of carbohydrates have the ability to regulate their carbohydrate intakes (i.e., to choose to eat amounts of each diet that, taken together, will give them a desired proportion of carbohydrate), and that this ability is independent of whether or not the carbohydrate consumed has a sweet taste. It is proposed that serotoninergic neurons comprise part of a behavioural feedback loop, whereby the consumption of carbohydrate (which, by altering plasma amino acid patterns, accelerates serotonin synthesis in brain neurons) diminishes the rat's subsequent tendency to consume additional carbohydrates. Drugs that enhance central serotoninergic transmission can probably be substituted for dietary carbohydrate to activate this behavioural loop.

### Summary of the Invention

The present invention is based on the discovery that administration of an agent which selectively enhances serotonin-mediated neurotransmission is useful in the treatment of disturbances of mood (e.g., depression, anxiety) and of appetite (e.g., carbohydrate craving, weight gain) commonly associated with the Premenstrual Syndrome (PMS). Agents or drugs useful in enhancing serotonin-mediated neurotransmission, or the effect of serotonin within the brain synapses, are referred to as serotoninergic drugs and include 1) drugs which act to increase the quantity of serotonin present within the synapses and 2) drugs which act to enhance the effects of serotonin present within brain synapses, generally by activating post-synaptic serotonin receptors.

Drugs which act to increase the quantity of serotonin within brain synapses include those which act to cause serotonin release, or suppress its uptake.

Related drugs, the serotonin agonists, share with these drugs the ability to enhance serotonin-mediated neurotransmission. The present invention relates to the use of drugs which cause serotonin release or suppress its uptake for the manufacture of a medicament for treating disturbances of mood, disturbances of appetite, or both, associated with premenstrual syndrome in women.

One or more of the serotoninergic drugs for the use of of the present invention can be administered to an individual in an amount effective to reduce or prevent the mood and/or appetite disturbances which would otherwise be observed in the individual prior to onset of menstruation. The drug (or drugs) can be administered, for example, orally, by subcutaneous, or other injection, intravenously, parenterally, transdermally, or rectally and can be given in various forms, such as a powder, tablet, capsule, solution or emulsion. In these various forms, the serotoninergic drug or drugs can be combined with additional substances, such as those needed to serve as fillers, diluents, binders, flavorings or coloring agents or coating materials.

The length of time during which a serotoninergic drug or drugs for the use of the present invention will be given varies on an individual basis, but will generally begin 1 to 14 days prior to menstruation and may continue for several days (e.g., 3 days) after onset of menstruation.

In one embodiment of the present invention, d-fenfluramine, or d,l-fenfluramine, which act to release serotonin and inhibit its inactivation by reuptake, is administered to an individual, prior to the onset of her menstrual period, in a quantity sufficient to ameliorate or prevent the mood disturbances and/or to suppress the weight gain and the increased appetite which otherwise would be evident. In a further embodiment, fluoxetine, which acts to inhibit uptake of serotonin, is administered in a quantity sufficient to suppress these effects.

The use of a serotoninergic drug according to the present invention is of great benefit to women who experience disturbances of mood and/or appetite prior to onset of their menstrual period because the drug or drugs administered act to alleviate or prevent such adverse premenstrual symptoms.

### Detailed Description of the Invention

The present invention relates to the use of one or more serotoninergic agents or drugs which cause serotonin release or suppress its uptake (i.e., one or more agents or drugs which selectively enhance serotonin-mediated neurotransmission) or the use of compositions comprising one or more of said serotoninergic agents or drugs for the manufacture of a medicament useful in alleviating or preventing disturbances of mood and/or appetite which occur prior to onset of menstruation, as well as in treating such disturbances.

Serotoninergic drugs included in compositions for the use of the present invention act to enhance serotonin-mediated neurotransmission by increasing the quantity of serotonin present within brain synapses, by activating post-synaptic serotonin receptors, or both. One or more of such serotoninergic drugs may be present in the composition and may be present alone (i.e., only serotoninergic drug(s)) or in combination with other substances which function in another capacity (e.g., as a filler, binder, etc.), as described below.

The neurotransmitter serotonin (5-hydroxytryptamine or 5-HT) is 3-(beta-aminoethyl)-5-hydroxyindole. It stimulates or inhibits a variety of smooth muscles and nerves and, among others, has effects on secretion by both exocrine and endocrine glands and on functioning of the respiratory, cardiovascular and central nervous systems. Within the central nervous system (CNS), serotonin serves as a neurotransmitter in the brain and spinal cord, where it is the chemical transmitter of neurons referred to as tryptaminergic or serotoninergic neurons. These neurons are involved in control of sleep, appetite, nutrient selection, blood pressure, mood, endocrine secretion, aggressivity and numerous other sensitivities to external stimuli.

Numerous substances or drugs have been shown to affect serotonin activity. For example, endogenous serotonin levels can be increased by administering tryptophan, the precursor of serotonin. Fernstrom, J.D. and Wurtman, R.J., Science, 173:149-152 (1971).

It has now been discovered that administration of an agent or a drug which selectively enhances serotonin-mediated neurotransmission by causing serotonin release or suppressing its uptake suppresses the weight gain and the increased appetite, particularly for carbohydrates, as well as decreasing the depression and other negative mood states, which many women experience prior to onset of menstruation. An agent or a drug which selectively enhances serotoninmediated neurotransmission has been shown to be particularly effective in having these effects.

Administration of a drug (or drugs) which enhances serotonin-mediated neurotransmission by increasing the quantity of serotonin within brain synapses or by activating post-synaptic serotonin receptors results in amelioration or elimination of these commonly-experienced adverse effects, but this is not a feature of the present claims.

For example, it has been shown that administration of d-fenfluramine (an anorectic drug) to women prior to onset of their menstrual period results in a decrease in depression and other negative mood states (e.g., tension, anger, confusion, irritability), as assessed using recognized tests (see Example 1) and in lower consumption of high-carbohydrate foods than observed when they were not given the drug (i.e., were given a placebo). A d-fenfluramine analogue, d,l-fenfluramine, has the same effect.

Similarly, administration of fluoxetine, which suppresses reuptake of serotonin and, thus, increases the quantity of serotonin available at brain synapses, has been shown to ameliorate the depressed moods and carbohydrate craving otherwise seen in subjects prior to their menstrual period. In addition, it was effective in suppressing the weight gain usually associated with the premenstrual phase in the subjects studied.

In addition to or, in the case of groups 2 an 3 below, instead of d-fenfluramine, d,l-fenfluramine and fluoxetine, other drugs which have the effect of enhancing serotonin-mediated neurotransmission can be administered. For example, the quantity of serotonin present at a given time or over a period of time can be enhanced by administering a drug which has any of the following effects:
1. increases serotonin production (e.g., tryptophan lithium);
2. causes serotonin release, e.g., d-fenfluramine, d,l-fenfluramine chlorimipramine (also known as clomipromine);
3. suppresses serotonin uptake, e.g., fluoxetine, fluvoxamine, citalopram, femoxetine, cianopramine, ORG 6582, RU 25591, LM5008, sertraline or 1S-4S-N-methyl-4-(3,4 dichlorophenyl)-1,2,3,4,-tetrahydro-1-naphthylamine, paroxetine, DU 24565, indalpine, CGP 6085/A, WY 25093, alaprociate, zimelidine, cyanimipramine, desyrel (trazodone hydrochloride) or trazodone amitriptyline or elavil (amitriptyline hydrochloride), imipramine or tofranil (imipramine hydrochloride), trimipramine or surmontil, doxepin or sinequan (doxepin hydrochloride), protriptyline or vivactil (protriptyline hydrochloride), nortriptyline or aventyl (nortriptyline hydrochloride), dibenzoxazepine (also known as amoxapine or asendin);
4. blocks presynaptic receptors, e.g., metergoline, methysergide, cyproheptadine (which can also block postsynaptic receptors); or
5. blocks monoamine oxidase, e.g., deprenyl, marplan or isocarboazide, nardil (phenelzine sulfate) or phenelzine, parnate (tranylcypromine sulfate) or tranylcypromine, furazolidone, procarbazine, moclobemide or aurorix, brofaromine).
The chemical names of DU 24565, CGP 6085/A, and WY 25093 are, respectively, 6-nitroquipazine, 4-(5,6-dimethyl-2-benzofuranyl) piperidine HCl, and 1-[1-([indol-3-yl]methyl) piperid-4-yl]-3-benzoylurea, respectively. Classen, K., et al., Naunyn Schmiedebergs Arch. Pharmacol., 326(3): 198-202 (1984); Kulakowski, E.C. et al., Clin. Exp. Hypertens. [A], 7(4): 585-604 (1985); Diggory, G.L. et al., Arch. Int. Pharacodyn. Ther., 248(1): 86-104 (1980).

The present invention relates to the use of drugs which fall into classes 2 and 3 above and the following example demonstrate the use thereof.

Alternatively, serotonin-mediated neurotransmission can be enhanced by administering a drug, such as quipazine, m-CPP, MK212 or CM57493, which activates post-synaptic serotonin receptors.

In either case, such agents or drugs can be administered individually or in combination. The quantity of an individual drug to be administered will be determined on an individual basis and will be based at least in part on consideration of the individual's size, the severity of symptoms to be treated and the result sought.

The agent(s) or drug(s) can be administered orally, by subcutaneous or other injection, intravenously, parenterally, transdermally, or rectally. The form in which the drug will be administered (e.g., powder, tablet, capsule, solution, emulsion) will depend on the route by which it is administered.

The composition for the use of the present invention can optionally include, in addition to the serotoninergic drug or drugs, other components. The components included in a particular composition are determined primarily by the manner in which the composition is to be administered. For example, a composition to be administered orally in tablet form can include, in addition to one or more serotoninergic drugs, a filler (e.g., lactose), a binder (e.g., carboxymethyl-cellulose, gum arabic, gelatin), an adjuvant, a flavoring agent, a coloring agent and a coating material (e.g., wax or a plasticizer). A composition to be administered orally, but in liquid form, can include one or more serotoninergic drugs, and, optionally, an emulsifying agent, a flavoring agent and/or a coloring agent.

In general, the composition for the use of the present invention is administered to an individual prior to the expected onset of her menstrual period. The length of time during which the drug (or drugs) is administered varies on an individual basis, but in general will be from 1 to 14 days prior to onset of menstruation and might continue (e.g., 3 days) after its onset. The dose of serotoninergic drug administered daily will also vary on an individual basis and to some extent will be determined by the type and severity of symptoms to be treated. If the serotoninergic drug administered is d-fenfluramine or d,l-fenfluramine, a dose of from approximately 7 mg/day to approximately 60 mg/day is administered. As described in Example I, a dose of 30 mg/day of d-fenfluramine has been shown to be effective in decreasing depression and other negative mood states in subjects. In the case of fluoxetine administration, a dose of from approximately 5 mg/day to approximately 120 mg/day is administered. As described in Example II, a dose of 40 mg/day, given on alternate days, has been shown to be effective in ameliorating the depressed mood and carbohydrate craving reported by subjects not given fluoxetine. It was also effective in suppressing the weight gain usually experienced. (See Example II). The serotoninergic drug can be administered in a single dose or in a number of smaller doses over a period of time; for example, the 30 mg/day dose of d-fenfluramine can be administered in a series of smaller doses over the course of the day.

The present invention will now be illustrated by the following examples, which are not to be taken as limiting in any way.

### Example I Assessment of effect of d-fenfluramine on Mood and Appetite Disturbances Associated with PMS

Seventeen women received either d-fenfluramine (30 mg/day) or a placebo for 15 days prior to their expected menstrual period. Each subject participated in 6 randomized test periods; in 3 of the test periods, each was given d-fenfluramine and in the other 3 test periods, was given a placebo. Mood was assessed 1-3 days before the onset of menses, using the Hamilton Depression Scale and the PMS Symptom Rating Scale, for mood and appetite symptoms. Hamilton, N., Journal of Neurosurgery and Psychiatry, 23:56-62 (1960); Steiner, M. et al., Acta Psychiatrica Scandinavia, 62:177-190 (1980). Food intake was measured through the use of self-reports (when subjects were out-patients), and directly (while subjects were inpatients), during one drug and one placebo period; subjects also were weighed. As shown in Table 1, 15 of the 17 patients reported a decrease in depression and other negative mood states (such as tension, anger, confusion, and irritability) following drug treatment, but not following placebo treatment.

**TABLE 1**

| Effect of D-fenfluramine on PMS Symptoms of Mood (Hamilton Depression Scale*) | | |
|---|---|---|
| Patient No. | Placebo | D-fenfluramine |
| 1 | 7 | 1 |
| 2 | 11 | 2 |
| 3 | 12 | 2 |
| 4 | 10 | 14 |
| 5 | 12 | 20 |
| 6 | 14 | 6 |
| 7 | 14 | 9 |
| 8 | 17 | 10 |
| 9 | 17 | 9 |
| 10 | 18 | 15 |
| 11 | 18 | 0 |
| 12 | 21 | 4 |
| 13 | 22 | 5 |
| 14 | 23 | 6 |
| 15 | 26 | 15 |
| 16 | 27 | 12 |
| 17 | 29 | 1 |
| Mean Score: | 18 | 8 |

| | | |
|---|---|---|
| *Higher scores on these tests indicate greater severity of symptoms. | | |

It was found that consumption of high carbohydrate foods increased for patients taking the placebo, but not for patients treated with d-fenfluramine. Appetite and mood (meausured by the "PMS Symptoms Checklist" described by Steiner et al., ibid.) were assessed 1-3 days before the onset of menses. The results are shown in Table 2, which reflect mean scores for eleven of the seventeen women tested:

**TABLE 2**

| Effect of D-fenfluramine on PMS Symmtoms of Mood and Appetite | | |
|---|---|---|
| Mood Scores (mean score) | Placebo | D-fenfluramine |
| PMS Sympt. Checklist | | |
| Mood | 38 | 0 |
| Appetite | 8 | 1 |

| Food Intake | | |
|---|---|---|
| Calories | 3300 | 1660 |
| CHO(g)* | 232 | 130 |
| Protein | 78 | 85 |
| (Higher scores on these tests indicate greater severity of symptoms) | | |

| | | |
|---|---|---|
| *CHO = carbohydrates | | |

### Example II Assessment of Effect of Fluoxetine on Mood and Appetite Disturbance Associated with PMS

Fluoxetine (40 mg/day) was given on alternate days, starting two weeks prior to the expected onset of a subject's menstrual period. Amelioration of the depressed mood and the carbohydrate cravings was reported (using the PMS Symptom Rating Scale): Mean scores for subjects taking the placebo were 36 and 10 (for mood and appetite, respectively), and 9 and 3 for subjects taking fluoxetine. Fluoxetine also suppressed the usual weight gain associated with the premenstrual phase in these particular subjects.

## Claims

1. Use of one or more serotonin-mediated neurotransmission enhancing drugs selected from the group comprising drugs which cause serotonin release and drugs which suppress serotonin uptake, for the manufacture of a medicament for treating disturbances of mood, appetite, or both, associated with premenstrual syndrome in women.

2. Use of a drug which causes serotonin release or a drug which blocks serotonin uptake for the manufacture of a medicament for treating disturbances of mood, disturbances of appetite, or both, associated with premenstrual syndrome, in a woman having premenstrual syndrome.

3. Use of d-fenfluramine, d,l-fenfluramine or chlorimipramine and a drug selected from the group consisting of fluoxetine, fluvoxamine, citalopram, femoxetine, paroxetine, ciamopramine, ORG 6582, RU 25591, LM 5008, 1S-4S-N-methyl-4-(3,4-dichlorophenyl)-1,2,3,4-tetrahydro-1-naphthylamine (sertraline), DU 24565, indalpine, CGP 6085/A, WY 25093, alaprociate, zimelidine, trazodone cyanimipramine, amitriptyline, imipramine, trimipramine, doxepin, protriptyline, and dibenzoxazepine; for the manufacture of a medicament for treating disturbances of mood, disturbances of appetite, or both, associated with premenstrual syndrome, in a woman having premenstrual syndrome.

4. Use of at least two serotoninergic drugs for the manufacture of a medicament for treating disturbances of mood, disturbances of appetite, or both, associated with premenstrual syndrome, in women, wherein each drug is selected from the group consisting of drugs which cause serotonin release and drugs which suppress serotonin uptake.

5. Use of a composition for the manufacture of a medicament for administration to women for treating disturbances of mood, disturbances of appetite, or both, associated with premenstrual syndrome, comprising at least two drugs which enhance serotonin-mediated neurotransmission being selected from the group consisting of drugs which cause serotonin release and drugs which suppress serotonin uptake.

6. Use of a composition for the manufacture of a medicament for treating disturbances of mood, disturbances of appetite, or both, associated with premenstrual syndrome, in a woman having premenstrual syndrome, comprising a drug which either causes serotonin release, or blocks serotonin uptake.

7. Use of a composition for the manufacture of a medicament for treating disturbances of mood, disturbances of appetite, or both, associated with premenstrual syndrome, in a woman having premenstrual syndrome, comprising d-fenfluramine, d,l-fenfluramine or chlorimipramine and a drug selected from the group consisting of fluoxetine, fluvoxamine, citalopram, femoxetine, paroxetine, ciamopramine, ORG 6582, RU 25591, LM 5008, 1S-4S-N-methyl-4-(3,4-dichlorophenyl)-1,2,3,4-tetrahydro-1-naphthylamine (sertraline), DU 24565, indalpine, CGP 6085/A, WY 25093, alaprociate, zimelidine, trazodone cyanimipramine, amitriptyline, imipramine, trimipramine, doxepin, protriptyline, and dibenzoxazepine.

8. Use of a composition for the manufacture of a medicament for administration to women for treating disturbances of mood, disturbances of appetite, or both, associated with premenstrual syndrome, comprising at least two serotoninergic drugs, wherein each drug is selected from the group consisting of drugs which cause serotonin release and drugs which suppress serotonin uptake.

9. Use of two or more serotonin-mediated neurotransmission enhancing drugs for the manufacture of a medicament for treating disturbances of mood, disturbances of appetite, or both, associated with premenstrual syndrome in women, wherein each drug is selected from the group consisting of drugs which cause serotonin release and drugs which suppress serotonin uptake.

10. Use according to any one of claims 1, 4, 5, 8 and 9, wherein the drugs are selected from the group consisting of d-fenfluramine, d,l-fenfluramine, chlorimipramine, cyanimipramine, fluoxetine, paroxetine, fluvoxamine, citalopram, femoxetine, cianopramine, ORG 6582, RU 25591, LM 5008, 1S-4S-N-methyl-4-(3,4-dichlorophenyl)-1,2,3,4-tetrahydro-1-naphthylamine (sertraline), DU 24565, indalpine, CGP 6085/A, WY 25093, alaprociate, zimelidine, trazodone, amitriptyline, imipramine, trimipramine, doxepin, protriptyline, nortriptyline, dibenzoxazepine, quipazine, m-CCP, MK212 and CM 57493.

11. Use according to any preceding claim, wherein the woman is treated prior to the onset of her menstrual period with either (a) 7 mg to 60 mg per day of d-fenfluramine or of d,l-fenfluramine; or (b) 5 mg to 120 mg of fluoxetine.

12. Use according to claim 11, wherein the woman is treated for 1 to 14 days prior to the onset of her menstrual period.

## Patentansprüche

1. Verwendung von einem oder weiteren die Serotonin-vermittelte Neurotransmission steigernden Wirkstoffen, ausgewählt aus der Gruppe Wirkstoffe zur Auslösung der Serotoninfreisetzung und Wirkstoffe zur Unterdrückung der Scrotoninaufnahme, zur Herstellung eines Arzneimittels zur Behandlung von mit dem prämenstruellen Syndrom bei Frauen assoziierten Störungen der Stimmung, des Appetits oder beiden.

2. Verwendung eines die Serotoninfreisetzung auslösenden Wirkstoffs oder eines die Serotoninaufnahme hemmenden Wirkstoffs zur Herstellung eines Arzneimittels zur Behandlung von mit dem prämenstruellen Syndrom assoziierten Stimmungsstörungen, Appetitstörungen oder beiden bei einer Frau mit dem prämenstruellen Syndrom.

3. Verwendung von d-Fenfluramin, d,l-Fenfluramin oder Chlorimipramin und einem Wirkstoff, ausgewählt aus der Gruppe Fluoxetin, Fluvoxamin, Citalopram, Femoxetin, Paroxetin, Cianopramin, ORG 6582, RU 25591, LM 5008, 1S-4S-N-Methyl-4-(3,4-dichlorphenyl)-1,2,3,4-tetrahydro-1-naphthylamin (Sertralin), DU 24565, Indalpin, CGP 6085/A, WY 25093, Alaprociat, Zimelidin, Trazodon, Cyanimipramin, Amitriptylin, Imipramin, Trimipramin, Doxepin, Protriptylin und Dibenzoxazepin, zur Herstellung eines Arzneimittels zur Behandlung von mit dem prämenstruellen Syndrom assoziierten Stimmungsstörungen, Appetitstörungen oder beiden bei einer Frau mit dem prämenstruellen Syndrom.

4. Verwendung von mindestens zwei serotoninergen Wirkstoffen zur Herstellung eines Arzneimittels zur Behandlung von mit dem prämenstruellen Syndrom bei Frauen assoziierten Stimmungsstörungen, Appetitstörungen oder beiden, wobei jeder Wirkstoff ausgewählt ist aus der Gruppe Wirkstoffe zur Auslösung der Serotoninfreisetzung und Wirkstoffe zur Unterdrückung der Serotoninaufnahme.

5. Verwendung einer Zusammensetzung zur Herstellung eines Arzneimittels zur Verabreichung an Frauen zur Behandlung von Stimmungsstörungen, Appetitstörungen oder beiden, die mit dem prämenstruellen Syndrom assoziiert sind, wobei die Zusammensetzung mindestens zwei die Serotonin-vermittelte Neurotransmission beschleunigende Wirkstoffe umfaßt, ausgewählt aus der Gruppe Wirkstoffe zur Auslösung der Serotoninfreisetzung und Wirkstoffe zur Unterdrückung der Serotoninaufnahme.

6. Verwendung einer Zusammensetzung zur Herstellung eines Arzneimittels zur Behandlung von mit dem prämenstruellen Syndrom assoziierten Stimmungsstörungen, Appetitstörungen oder beiden bei einer Frau mit dem prämenstruellen Syndrom, wobei die Zusammensetzung einen Wirkstoff umfaßt, der entweder die Serotoninfreisetzung bewirkt oder die Serotoninaufnahme hemmt.

7. Verwendung einer Zusammensetzung zur Herstellung eines Arzneimittels zur Behandlung von mit dem prämenstruellen Syndrom assoziierten Stimmungsstörungen, Appetitstörungen oder beiden bei einer Frau mit dem prämenstruellen Syndrom, wobei die Zusammensetzung d-Fenfluramin, d,l-Fenfluramin oder Chlorimipramin und einen Wirkstoff umfaßt, der ausgewählt ist aus der Gruppe Fluoxetin, Fluvoxamin, Citalopram, Femoxetin, Paroxetin, Cianopramin, ORG 6582, RU 25591, LM 5008, 1S-4S-N-Methyl-4-(3,4-dichlorphenyl)-1,2,3,4-tetrahydro-1-naphthylamin (Sertralin), DU 24565, Indalpin, CGP 6085/A, WY 25093, Alaprociat, Zimelidin, Trazodon, Cyanimipramin, Amitriptylin, Imipramin, Trimipramin, Doxepin, Protriprylin und Diberzoxazepin.

8. Verwendung einer Zusammensetzung zur Herstellung eines Arzneimittels zur Verabreichung an Frauen zur Behandlung von Stimmungsstörungen, Appetitstörungen oder beiden, die mit dem prämenstruellen Syndrom assoziiert sind, wobei die Zusammensetzung mindestens zwei serotoninerge Wirkstoffe umfaßt, wobei jeder Wirkstoff ausgewählt ist aus der Gruppe Wirkstoffe zur Auslösung der Serotoninfreisetzung und Wirkstoffe zur Unterdrückung der Serotoninaufnahme.

9. Verwendung von bei oder mehr die Serotonin-vermittelte Neurotransmission steigernden Wirkstoffen zur Herstellung eines Arzneimittels zur Behandlung von mit dem prämenstruellen Syndrom bei Frauen assoziierten Stimmungsstörungen, Appetitstörungen oder beiden, wobei jeder Wirkstoff ausgewählt ist aus der Gruppe Wirkstoffe zur Auslösung der Serotoninfreisetzung und Wirkstoffe zur Unterdrückung der Serotoninaufnahme.

10. Verwendung nach jedern der Ansprüche 1, 4, 5, 8 und 9, wobei die Wirkstoffe ausgewählt sind aus der Gruppe d-Fenfluramin, d,l-Fenfluramin, Chlorimipramin, Cyanimipramin, Fluoxetin, Paroxetin, Fluvoxamin, Citalopram, Femoxetin, Cianopramin, ORG 6582, RU 25591, LM 5008, 1S-4S-N-Methyl-4-(3,4-dichlorphenyl)-1,2,3,4-tetrahydro-1-naphthylamin (Sertraiin), DU 24565, Indalpin, CGP 6085/A, WY 25093, Alaprociat, Zimelidin, Trazodon, Amitriptylin, Imipramin, Trimipramin, Doxepin, Protriptylin, Nortriprylin, Dibenzoxazepin, Quipazin, m-CCP, MK 212 und CM 57493.

11. Verwendung nach jedem der vorhergehenden Ansprüche, wobei die Frau vor Beginn der Menstruationsperiode entweder mit (a) 7 bis 60 mg d-Fenfluramin oder d,l-Fenfluramin pro Tag oder mit (b) 5 bis 120 mg Fluoxetin behandelt wird.

12. Verwendung nach Anspruch 11, wobei die Frau 1 bis 14 Tage vor Beginn der Menstruationsperiode behandelt wird.

## Revendications

1. Utilisation d'un ou plusieurs produits stimulant la neurotransmission médiée par la sérotonine choisis parmi des produits qui provoquent une libération de sérotonine et des produits qui suppriment l'absorption de sérotonine, pour la fabrication d'un médicament pour le traitement de troubles de l'humeur, de l'appétit ou des deux, associés au syndrome prémenstruel chez les femmes.

2. Utilisation d'un produit qui provoque la libération de sérotonine ou d'un produit oui bloque l'absorption de sérotonine pour la fabrication d'un médicament pour le traitement de troubles de l'humeur, troubles de l'appétit, ou les deux, associés au syndrome prémenstruel, chez une femme présentant le syndrome prémenstruel.

3. Utilisation de d-fenfluramine, de d,l-fenfluramine ou de chlorimipramine et d'un produit choisi dans l'ensemble constitué de fluoxétine, fluvoxamine, citalopram, fémoxétine, paroxétine, ciamopramine, ORG 6582, RU 25591, LM 5008, 1S-4S-N-méthyl-4-(3,4-dichlorophényl)-1,2,3,4-tétrahydro-1-naphtylamine (sertraline), DU 24565, indalpine, CGP 6085/A, WY 25093, alaprociate, zimélidine, trazodone, cyanimipramine, amitriptyline, imipramine, trimipramine, doxépine, protriptyline, et dibenzoxazépine, pour la fabrication d'un médicament pour le traitement de troubles de l'humeur, troubles de l'appétit, ou les deux, associés au syndrome prémenstruel, chez une femme présentant le syndrome prémenstruel.

4. Utilisation d'au moins deux produits sérotoninergiques pour la fabrication d'un médicament pour le traitement de troubles de l'humeur, troubles de l'appétit, ou les deux, associés au syndrome prémenstruel, chez les femmes, chaque produit étant choisi dans l'ensemble constitué de produits oui provoquent une libération de sérotonine et de produits qui suppriment l'absorption de sérotonine.

5. Utilisation d'une composition pour la fabrication d'un médicament destiné à être administré à des femmes pour le traitement de troubles de l'humeur, troubles de l'appétit, ou les deux, associés au syndrome prémenstruel, comprenant au moins deux produits qui stimulent la neurotransmission à médiation par la sérotonine, étant choisis dans l'ensemble constitué de produits qui provoquent une libération de sérotonine et de produits oui suppriment l'absorption de sérotonine.

6. Utilisation d'une composition pour la fabrication d'un médicament pour le traitement de troubles de l'humeur, troubles de l'appétit ou des deux, associés au syndrome prémenstruel chez une femme présentant un syndrome prémenstruel, comprenant un produit oui provoque une libération de sérotonine ou bloque l'absorption de sérotonine.

7. Utilisation d'une composition pour la fabrication d'un médicament pour le traitement de troubles de l'humeur, troubles de l'appétit ou des deux, associés au syndrome prémenstruel, chez une femme présentant un syndrome prémenstruel, comprenant de la d-fenfluramine, de la d,l-fenfluramine ou de la chlorimipramine et un produit choisi dans l'ensemble constitué de fluoxétine, fluvoxamine, citalopram, fémoxétine, paroxétine, ciamopramine, ORG 6582, RU 25591, LM 5008, 1S-4S-N-méthyl-4-(3,4-dichlorophényl)-1,2,3,4-tétrahydro-1-naphtylamine (sertraline), DU 24565, indalpine, CGP 6085/A, WY 25093, alaprociate, zimélidine, trazodone, cyanimipramine, amitriptyline, imipramine, trimipramine, doxépine, protriptyline, et dibenzoxazépine.

8. Utilisation d'une composition pour la fabrication d'un médicament destiné à être administré à des femmes pour le traitement de troubles de l'humeur, troubles de l'appétit ou des deux, associés au syndrome prémenstruel, comprenant au moins deux produits sérotoninergioues, chaque produit étant choisi dans l'ensemble constitué de produits qui provoquent une libération de sérotonine et de produits qui suppriment l'absorption de sérotonine.

9. Utilisation de deux produits activant la neurotransmission à médiation par la sérotonine ou plus, pour la fabrication d'un médicament pour le traitement de troubles de l'humeur, troubles de l'appétit, ou les deux, associés au syndrome prémenstruel chez les femmes, chaque produit étant choisi dans l'ensemble constitué de produits oui provoquent une libération de sérotonine et de produits qui suppriment l'absorption de sérotonine.

10. Utilisation selon l'une quelconque des revendications 1, 4, 5, 8 et 9, dans laquelle les produits sont choisis dans l'ensemble constitué de d-fenfluramine, d,l-fenfluramine, chlorimipramine, cyanimipramine, fluoxétine, paroxétine, fluvoxamine, citalopram, fémoxétine, cianopramine, ORG 6582, RU 25591, LM 5008, 1S-4S-N-méthyl-4-(3,4-dichlorophényl)-1,2,3,4-tétrahydro-1-naphtylamine (sertraline), DU 24565, indalpine, CGP 6085/A, WY 25093, alaprociate, zimélidine, trazodone, amitriptyline, imipramine, trimipramine, doxépine, protriptyline, nortryptiline, dibenzoxazépine, quizapine, m-CCP, MK212 et CM 57493.

11. Utilisation selon l'une quelconque des revendications précédentes, dans laquelle la femme est traitée avant le début de sa période menstruelle avec (a) 7 mg à 60 mg par jour de d-fenfluramine ou de d,l-fenfluramine; ou bien (b) 5 mg à 120 mg de fluoxétine.

12. Utilisation selon la revendication 11, dans laquelle la femme est traitée pendant 1 à 14 jours avant le début de sa période menstruelle.
